(19) Europäisches Patentamt
European Patent Office
Office européen des brevets

(11) Veröffentlichungsnummer: **0 370 382 A2**

# (12) EUROPÄISCHE PATENTANMELDUNG

(21) Anmeldenummer: **89121189.8**

(22) Anmeldetag: **16.11.89**

(51) Int. Cl.5: **C07K 5/02, C07C 275/12, A61K 37/64**

Claims for the following Contracting States: ES + GR.

(30) Priorität: **19.11.88 DE 3839126**

(43) Veröffentlichungstag der Anmeldung: **30.05.90 Patentblatt 90/22**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Rüger, Wolfgang, Dr.**
**Parkstrasse 10**
**D-6233 Kelkheim (Taunus)(DE)**
Erfinder: **Urbach, Hansjörg, Dr.**
**Le Lavandoustrasse 41**
**D-6242 Kronberg/Taunus(DE)**
Erfinder: **Ruppert, Dieter, Dr.**
**Schreyerstrasse 30**
**D-6242 Kronberg/Taunus(DE)**
Erfinder: **Schölkens, Bernward, Prof. Dr.**
**Hölderlinstrasse 62**
**D-6233 Kelkheim (Taunus)(DE)**

## (54) Renin-hemmende Harnstoffderivate von Dipeptiden, Verfahren zu deren Herstellung, diese enthaltende Mittel und ihre Verwendung.

(57) Die vorliegende Erfindung betrifft Verbindungen der Formel I

$$R^1 - A - B - HN - \overset{\overset{\large R^2}{|}}{CH} - \overset{\overset{\large OH}{|}}{CH} - \overset{\overset{\large R^3}{|}}{CH} - R^4 \qquad (I)$$

in welcher
A und B unabhängig voneinander den Rest einer Aminosäure bedeuten und
$R^1$ bis $R^4$ wie in der Beschreibung angegeben definiert sind,
Verfahren zu ihrer Herstellung, diese enthaltende Mittel und ihre Verwendung.

EP 0 370 382 A2

## Renin-hemmende Harnstoffderivate von Dipeptiden, Verfahren zu deren Herstellung, diese enthaltende Mittel und ihre Verwendung

Aus den EP-A-172 346, EP-A-172 347, EP-A-189 203, EP-A-229 667, EP-A-230 266, EP-A-255 082, EP-A 273 893 und EP-A-274 259 sind Dipeptidderivate und deren Verwendung als Renin-Inhibitoren bekannt.

Es wurden nun neue Harnstoffderivate von Dipeptiden gefunden, die in vitro und in vivo hochwirksam das Enzym Renin hemmen, sowie ein neues Verfahren zur Herstellung dieser Verbindungen.

Die Erfindung betrifft Verbindungen der Formel I

$$R^1 - A - B - HN - \overset{R^2}{\overset{|}{CH}} - \overset{OH}{\overset{|}{CH}} - \overset{R^3}{\overset{|}{CH}} - R^4 \qquad (I)$$

in welcher

$R^1$ einen Rest der Formel II bedeutet

$R^a$ - NH - CO - (II)

worin

$R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,

($C_3$-$C_8$)-Cycloalkyl, ($C_3$-$C_8$)-Cycloalkyl-($C_1$-$C_6$)-alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,

($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist,

A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin und (E)-Dehydrophenylalanin bedeutet,

B einen N-terminal mit A und C-terminal mit

$$NH - \overset{R^2}{\overset{|}{CH}} - \overset{OH}{\overset{|}{CH}} - \overset{R^3}{\overset{|}{CH}} - R^4$$

verknüpften Rest einer Aminosäure, der wie unter A definiert ist, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

$R^4$ einen Rest der Formel III bedeutet

$-(CH_2)_p-X-(CH_2)_q-R^5$ (III)

worin X wahlweise abwesend sein kann oder für -O-, -S-, $-CF_2-$, -CO- oder $-CHR^6-$ steht,

p und q unabhängig voneinander 0, 1, 2, 3 oder 4 bedeuten,

$R^6$ Wasserstoff, $(C_1-C_7)$-Alkyl, $(C_1-C_5)$-Alkoxy, $(C_1-C_5)$-Alkylthio, $(C_1-C_5)$-Alkylamino-, -OH, $-N_3$, -F, -Cl, -Br oder -I bedeutet, und

$R^5$ Wasserstoff, -OH, $-NH_2$ oder Heteroaryl, das auch teilweise oder vollständig hydriert sein kann, bedeutet sowie deren physiologisch verträgliche Salze.

Die durch $R^2$, $R^3$ und $R^6$ substituierten C-Atome können jeweils die R-, S- oder R,S-Konfiguration besitzen.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit $(C_1-C_6)$-Alkyl verknüpften unsubstituierten oder substituierten $(C_6-C_{14})$-Aryl-Rest, wie z.B. Benzyl, α- und ß-Naphthylmethyl, Halobenzyl und Alkoxybenzyl, wobei Aralkyl jedoch nicht auf die genannten Reste beschränkt wäre.

Unter einem Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom als Ringglieder werden Reste von Heteroaromaten verstanden, wie sie beispielsweise in Katritzky, Lagowski, Chemie der Heterocyclen, Berlin, Heidelberg 1968, Seite 3 - 5 definiert sind. Der Heteroaromaten-Rest kann durch einen, zwei oder drei, vorzugsweise einer oder zwei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, Heteroaryloxy, Heteroaryl und Heteroaryl-alkyl.

Die Aminosäuren A und B in Formel I sind durch eine Amidbindung miteinander verknüpft, es handelt sich um natürliche oder nichtnatürliche α-Aminosäuren der L-, D-oder D,L-Konfiguration, vorzugsweise der L-Konfiguration.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)-amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt sind Verbindungen der Formel I, in denen

$R^1$ einen Rest der Formel II bedeutet, worin

$R^a$ Wasserstoff, $(C_1-C_{10})$-Alkyl, $(C_1-C_4)$-Alkoxy-$(C_1-C_{10})$-alkyl, Carboxy-$(C_1-C_{10})$-alkyl, $(C_1-C_4)$-Alkoxycarbonyl-$(C_1-C_{10})$-alkyl, Amino-$(C_1-C_{10})$-alkyl, Diamino-$(C_1-C_{10})$-alkyl, $(C_1-C_4)$-Alkylamino-$(C_1-C_{10})$-alkyl, Di$(C_1-C_4)$alkylamino-$(C_1-C_{10})$-alkyl, N,N′-Di-$(C_1-C_4)$-alkyldiamino-$(C_1-C_{10})$-alkyl, N,N,N′,N′-Tetra$(C_1-C_4)$-alkyldiamino-$(C_1-C_{10})$-alkyl, $(C_1-C_4)$-Alkoxycarbonylamino-$(C_1-C_{10})$-alkyl, $(C_7-C_{15})$-Aralkoxycarbonylamino-$(C_1-C_{10})$-alkyl, Cyclopentyl, Cyclohexyl, Cycloheptyl, Cyclopentyl-$(C_1-C_6)$-alkyl, Cyclohexyl-$(C_1-C_6)$-alkyl, Phenyl, das gegebenenfalls durch einen oder zwei gleiche oder verschiedene Reste aus der Reihe F, Cl, Br, J, Hydroxy, $(C_1-C_4)$-Alkoxy, $(C_1-C_4)$-Alkyl, $(C_1-C_4)$-Alkoxycarbonyl, Amino oder Trifluormethyl substituiert ist, $(C_6-C_{10})$-Aryl-$(C_1-C_6)$-alkyl, worin der Arylteil wie oben Phenyl substituiert sein kann, bedeutet oder für den Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist,

A einen N-terminal mit $R^1$ und C-terminal mit B verknüpften Rest einer Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin oder (E)-Dehydrophenylalanin bedeutet,

B einen N-terminal mit A und C-terminal mit

$$-NH-\underset{|}{\overset{R^2}{CH}}-\underset{|}{\overset{OH}{CH}}-\underset{|}{\overset{R^3}{CH}}-R^4$$

verknüpften Rest einer Aminosäure aus der Reihe

Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]-thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin und (E)-Dehydrophenylalanin, bedeutet,

$R^2$ Wasserstoff, $(C_1\text{-}C_{10})$-Alkyl, $(C_4\text{-}C_7)$-Cycloalkyl, $(C_4\text{-}C_7)$-Cycloalkyl-$(C_1\text{-}C_4)$-alkyl, $(C_6\text{-}C_{14})$-Aryl oder $(C_6\text{-}C_{14})$-alkyl bedeutet,

$R^3$ Wasserstoff, $(C_1\text{-}C_{10})$-Alkyl, $(C_6\text{-}C_{14})$-Aryl oder $(C_6\text{-}C_{14})$-Aryl-$(C_1\text{-}C_4)$-alkyl bedeutet,

$R^4$ einen Rest der Formel III bedeutet, worin

X wahlweise abwesend sein kann oder für -O-, -S-, $-CF_2-$, -CO- oder $-CHR^6-$ steht,

p und q unabhängig voneinander 0, 1, 2, 3 oder 4 bedeuten,

$R^6$ Wasserstoff, $(C_1\text{-}C_7)$-Alkyl, $(C_1\text{-}C_5)$-Alkoxy, $(C_1\text{-}C_5)$-Alkylthio, $(C_1\text{-}C_5)$-Alkylamino, -OH, $-N_3$, -F, -Cl, -Br oder -I bedeutet, und

$R^5$ Wasserstoff, -OH, $-NH_2$ oder Heteroaryl, das auch teilweise oder vollständig hydriert sein kann, bedeutet sowie deren physiologisch verträgliche Salze.

Insbesondere sind bevorzugt Verbindungen der Formel I, in denen

$R^1$ einen Rest der Formel II bedeutet, worin

$R^a$ Wasserstoff, $(C_1\text{-}C_6)$- Alkyl, Carboxy-$(C_1\text{-}C_6)$-alkyl, Amino-$(C_1\text{-}C_6)$-alkyl, Diamino-$(C_1\text{-}C_6)$-alkyl, $(C_1\text{-}C_4)$-Alkylamino-$(C_1\text{-}C_6)$-alkyl, Di-$(C_1\text{-}C_4)$-alkylamino-$(C_1\text{-}C_6)$-alkyl, N,N′-Di-$(C_1\text{-}C_4)$-alkyldiamino-$(C_1\text{-}C_6)$-alkyl, N,N,N′,N′-Tetra-$(C_1\text{-}C_4)$-alkyldiamino-$(C_1\text{-}C_6)$-alkyl, $(C_1\text{-}C_4)$-Alkoxycarbonylamino-$(C_1\text{-}C_6)$-alkyl oder Phenyl, das gegebenenfalls durch einen oder zwei gleiche oder verschiedene Reste aus der Reihe F, Cl, Hydroxy oder Amino substituiert ist, bedeutet,

A einen N-terminal mit $R^1$ und C-terminal mit B verknüpften Rest einer Aminosäure aus der Reihe Phenylalanin, Tyrosin, ß-2-Thienylalanin, ß-3-Thienylalanin, 4-Chlorphenylalanin, 0-Methyltyrosin, 0-Benzyltyrosin, 1-Naphthylalanin, 2-Fluorphenylalanin, 3-Fluorphenylalanin oder 4-Fluorphenylalanin bedeutet,

B einen N-terminal mit A und C-terminal mit

$$-NH-\underset{|}{\overset{R^2}{CH}}-\underset{|}{\overset{OH}{CH}}-\underset{|}{\overset{R^3}{CH}}-R^4$$

verknüpften Rest einer Aminosäure aus der Reihe

Phenylalanin, Histidin, Leucin, Asparagin, ß-2-Thienylalanin, ß-3-Thienylalanin, Lysin, Norvalin, 2-Fluorphenylalanin, 3-Fluorphenylalanin, 4-Fluorphenylalanin, Norleucin, S-Methylcystein oder N-Methylhistidin bedeutet,

$R^2$ Isobutyl, Cyclohexylmethyl oder Benzyl bedeutet,

$R^3$ Wasserstoff ist und

$R^4$ für einen Rest der Formel III steht, worin

X abwesend ist,

p 0 ist,

q 0, 1, 2, 3 oder 4 bedeutet und
$R^5$ 2-Pyridyl, 4-Pyridyl-, 2-Imidazolyl oder 1-($C_1$-$C_4$-Alkyl)-2-imidazolyl bedeutet
sowie deren physiologisch verträglichen Salze.

Die Erfindung betrifft ferner ein Verfahren zur Herstellung von Verbindungen der Formel I, das dadurch gekennzeichnet ist, daß man eine Verbindung der allgemeinen Formel IV

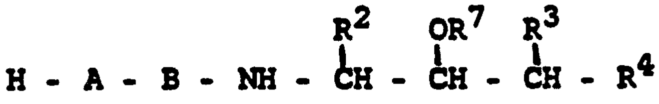

(IV)

in welcher A, B, $R^2$, $R^3$ und $R^4$ die gleiche Bedeutung wie in der allgemeinen Formel I besitzen und in welcher $R^7$ Wasserstoff oder eine leicht abspaltbare Schutzgruppe wie z.B. Methoxymethyl, Methylthiomethyl, Benzyloxymethyl, Tetrahydropyranyl, tert. Butyl, Allyl, Benzyl oder substituiertes Benzyl, Benzhydryl, Trityl, Trimethylsilyl, tert. Butyldimethylsilyl, Acetyl, Pivaloyl, Benzoyl, Methyloxycarbonyl, Ethyloxycarbonyl, Benzyloxycarbonyl bedeutet,

a) mit einem Isocyanat der allgemeinen Formel V

$R^a - N = C = O$ (V),

worin $R^a$ die gleiche Bedeutung wie in der allgemeinen Formel II besitzt, in einem geeigneten organischen Lösungsmittel wie z.B. Benzol, Toluol, Chlorbenzol, einem aliphatischen Kohlenwasserstoff, einem aliphatischen Chlorkohlenwasserstoff, Diethylether, Tetrahydrofuran, Dioxan, Aceton, Acetonitril, Dimethylformamid, Dimethylsulfoxid, oder aber unter Verzicht auf ein Lösungsmittel jeweils wahlweise mit oder ohne Zusatz einer Lewis-Säure oder einer Lewis-Base als Katalysator, wie z.B. eines tert.-Amins, vorzugsweise Triethylamin, 1,4-Diazabicyclo[2.2.2]octan, Cyclohexyldimethylamin, Benzyldimethylamin, 4-Methylmorpholin, Tetramethylguanidin oder 4-Dimethylaminopyridin, bei einer Temperatur zwischen -80 °C und der Siedetemperatur des Lösungsmittels, vorzugsweise zwischen -30 °C und +80 °C, umsetzt und gegebenenfalls die Schutzgruppe $R^7$ wieder abspaltet, oder

b) nacheinander zunächst mit einem Kohlensäurederivat der allgemeinen Formel VI

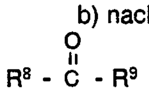

(VI),

worin $R^8$ und $R^9$ unabhängig voneinander Halogen, ($C_1$-$C_7$)-Alkoxy, ($C_6$-$C_{12}$)-Aryloxy, ($C_1$-$C_7$)-Alkylthio, ($C_6$-$C_{12}$)-Arylthio oder einen Rest Het oder Het-O-bedeuten, wobei Het ein mono- oder bicyclischer Heterocyclus sein kann, oder worin $R^8$ und $R^9$ gemeinsam mit der C=O-Gruppe einem mono- oder bicyclischen Heterocyclus vom Typ

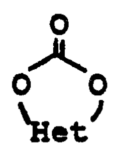

oder

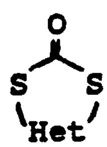

angehören,

vorzugsweise mit Phosgen, 1,1′-Carbonyldiimidazol, 1,1′-Carbonyldi-(1,2,4)-triazol, Di-(N-succinimidyl)-carbonat, Di-(1-benzotriazolyl)-carbonat, N,N′-Carbonyl-bis-(2-methylimidazol) oder 4,6-Diphenylthieno[3,4-d]-1,3-dioxol-2-on-5,5-dioxid (Steglich-Reagens) und anschließend mit einem Amin der allgemeinen Formel VII

$R^a - NH_2$ (VII),

worin $R^a$ die gleiche Bedeutung wie in der allgemeinen Formel II besitzt, in einem inerten organischen Lösungsmittel, vorzugsweise Toluol, Methylenchlorid, Chloroform, Tetrahydrofuran, Dimethylformamid oder Acetonitril, mit oder ohne Gegenwart einer Hilfsbase wie z.B. Kaliumcarbonat, Natriumcarbonat, Triethylamin, Pyridin, 1,5-Diazabicyclo[5.4.0]-undec-5-en oder 1,5-Diazabicyclo[4.3.0]-non-5-en, vorzugsweise Pyridin, bei einer Temperatur zwischen -80 °C und dem Siedepunkt des jeweiligen Lösungsmittels, vorzugsweise zwischen -80 °C und +50 °C umsetzt und gegebenenfalls nach bekannten Methoden die Schutzgruppe $R^7$ sowie gegebenenfalls in den Fragmenten A und B temporär eingeführte Schutzgruppen wieder abspaltet.

Die Verbindungen der allgemeinen Formel IV werden erhalten aus Verbindungen der allgemeinen Formel VIII,

$$R^{10} - A - B - NH - \underset{}{\overset{R^2}{\overset{|}{CH}}} - \overset{OH}{\overset{|}{CH}} - \overset{R^3}{\overset{|}{CH}} - R^4 \qquad (VIII)$$

worin $R^2$, $R^3$, $R^4$, A und B die gleiche Bedeutung wie in Formel I besitzen und worin $R^{10}$ eine leicht abzuspaltende Amino-Schutzgruppe bedeutet, vorzugsweise tert. Butyloxycarbonyl oder Benzyloxycarbonyl, durch Abspaltung dieser Schutzgruppe unter den üblichen Bedingungen, z.B. durch saure oder alkalische Hydrolyse oder Hydrogenolyse, gegebenenfalls unter vorherigem oder nachfolgendem Schutz der Hydroxyfunktion mit einem der üblichen zur Einführung des Restes $R^7$ geeigneten Reagenzien wie z.B. Methoxymethylchlorid, Methylthiomethylchlorid, Benzyloxymethylchlorid, Dihydropyran, Isobuten, Allylbromid, Benzylbromid, Diphenyldiazomethan, Tritylchlorid, Trimethylsilylchlorid, tert.-Butyldimethylsilylchlorid, Acetanhydrid, Pivaloylchlorid, Benzoylchlorid, Chlorameisensäuremethylester, Chlorameisensäureethylester oder Chlorameisensäurebenzylester.

Die Verbindungen der Formel V, VI und VII sind literaturbekannt und größtenteils käuflich. Die Verbindungen der Formel VIII sind aus der EP-A-255 082 bekannt.

Aus der EP-A-255 082 ist außerdem ein Verfahren zur Herstellung der Verbindungen der Formel I bekannt, das dadurch gekennzeichnet ist, daß man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppen(n) abspaltet und die so erhaltene Verbindung gegebenenfalls in ihr physiologisch verträgliches Salz überführt.

Das erfindungsgemäße Verfahren unterscheidet sich jedoch in mehrfacher Hinsicht vorteilhaft von dem herkömmlichen Verfahren:

A) Bei den herkömmlichen Verfahren sind im Gegensatz zu dem erfindungsgemäßen Verfahren zwei zusätzliche Reaktionsschritte bei der Einführung des Restes $R^1$, bedingt durch die Einführung und Abspaltung der Schutzgruppe am C-terminalen Ende der Aminosäure A-OH bzw. des Dipeptids A-B-OH, erforderlich. Bei dem erfindungsgemäßen Verfahren erfolgt die Einführung des Restes $R^1$ auf der letzten Stufe am ungeschützten N-Terminus des Dipeptids durch Umsetzung mit Isocyanaten bzw. Aminen, die außerdem in großer Anzahl und Variationsbreite zur Verfügung stehen.

B) Reste $R^1$, die eine zusätzliche Aminofunktion enthalten, können nach dem erfindungsgemäßen Verfahren in Form des Amins $R^a$-$NH_2$ (Formel VII) in ungeschützter Form direkt eingeführt werden (siehe Beispiel 13), wohingegen nach dem herkömmlichen Verfahren diese zusätzliche Aminogruppe zunächst geschützt und anschließend wieder entblockiert werden muß.

C) Das Auftreten von Nebenreaktionen wird bei dem erfindungsgemäßen Verfahren durch die Abwesenheit von Kupplungshilfsmitteln auf der letzten Stufe der Reaktionssequenz und die fehlende Aktivierung der C-terminalen Carboxylfunktion vermieden.

Die erfindungsgemäßen Verbindungen der Formel I weisen enzymhemmende Eigenschaften auf; insbesondere hemmen sie die Wirkung des natürlichen Enzyms Renin. Renin ist ein proteolytisches Enzym aus der Klasse der Aspartyl-Proteasen, welches als Folge verschiedener Stimuli (Volumendepletion, Natriummangel, ß-Rezeptorenstimulation) von den juxtaglomerulären Zellen der Niere in den Blutkreislauf sezerniert wird. Dort spaltet es von dem aus der Leber ausgeschiedenen Angiotensinogen das Decapeptid Angiotensin I ab. Dieses wird durch das "angiotensin converting enzyme" (ACE) in Angiotensin II überführt. Angiotensin II spielt eine wesentliche Rolle bei der Blutdruckregulation, da es direkt den Blutdruck durch Gefäßkontraktion steigert. Zusätzlich stimuliert es die Sekretion von Aldosteron aus der Nebenniere und erhöht auf diese Weise über die Hemmung der Natrium-Ausscheidung das extrazelluläre Flüssigkeitsvolumen, was seinerseits zu einer Blutdrucksteigerung beiträgt. Hemmer der enzymatischen Aktivität des Renins bewirken eine verminderte Bildung von Angiotensin I, was eine verminderte Bildung von Angiotensin II zur Folge hat. Die Erniedrigung der Konzentration dieses aktiven Peptidhormons ist die direkte Ursache für die blutdrucksenkende Wirkung von Renin-Hemmern.

Die Wirksamkeit von Renin-Hemmern kann durch in-vitro-Tests überprüft werden. Hierbei wird die Verminderung der Bildung von Angiotensin I in verschiedenen Systemen (Humanplasma, gereinigtes Humanrenin) gemessen.

## 1. Testprinzip

Z.B. Humanplasma, welches sowohl Renin als auch Angiotensinogen enthält, wird bei 37°C mit der zu testenden Verbindung inkubiert. Dabei wird aus Angiotensinogen unter der Einwirkung von Renin Angiotensin I freigesetzt, das anschließend mit einem handelsüblichen Radioimmunoassay gemessen werden kann. Diese Angiotensin-Freisetzung wird durch Renin-Inhibitoren gehemmt.

**2. Gewinnung des Plasmas**

Das Blut wird von freiwilligen Probanden gewonnen (ca. 0,5 l pro Person; Bluko-Entnahmegerät der Fa. ASID Bonz und Sohn, Unterschleißheim) und in teilweise evakuierten Flaschen unter Eiskühlung aufgefangen. Die Gerinnung wird durch Zugabe von EDTA (Endkonzentration 10 mM) verhindert. Nach dem Zentrifugieren (Rotor HS 4 (Sorvall), 3 500 Upm, 0-4° C, 15 min; wiederholen, falls erforderlich) wird das Plasma vorsichtig abpipettiert und in geeigneten Portionen bei -30° C eingefroren. Für den Test werden nur Plasmen mit ausreichend hoher Reninaktivität verwendet. Plasmen mit niedriger Reninaktivität werden durch eine Kältebehandlung (-4° C, 3 Tage) aktiviert (Prorenin → Renin).

**3. Durchführung des Tests**

Angiotensin I wird mit dem Renin-Maia®-Kit (Serono Diagnostics S.A., Coinsins, Schweiz) bestimmt. Die Inkubation des Plasmas wird nach der dort angegebenen Anleitung durchgeführt:

Inkubationsansatz:
1000 µl Plasma (bei 0-4° C aufgetaut)
100 µl Phosphatpuffer (pH 7,4) Zusatz von $10^{-4}$ M Ramiprilat)
10 µl PMSF-Lösung
10 µl 0,1 % Genapol PFIC
12 µl DMSO bzw. Testpräparat

Die Testpräparate werden i.a. $10^{-2}$ M in 100 % Dimethylsulfoxid (DMSO) gelöst und mit DMSO entsprechend verdünnt; der Inkubationsansatz enthält max. 1 % DMSO.

Die Ansätze werden in Eis gemischt und für 1 Stunde zur Inkubation in ein Wasserbad (37° C) gestellt. Aus einem zusätzlichen Ansatz ohne Inhibitor werden ohne weitere Inkubation insgesamt 6 Proben (jeweils 100 µl) zur Bestimmung des Ausgangs-Angiotensin I-Gehaltes des verwendeten Plasmas entnommen.

Die Konzentrationen der Testpräparate werden so gewählt, daß etwa der Bereich von 10-90 % Enzymhemmung abgedeckt ist (mindestens fünf Konzentrationen). Am Ende der Inkubationszeit werden aus jedem Ansatz drei 100 µl-Proben in vorgekühlten Eppendorf-Gefäßen auf Trockeneis eingefroren und bei ca. -25° C für die Angiotensin I-Bestimmung aufbewahrt (Mittelwert aus drei Einzelproben).

**Angiotensin I-Radioimmunoassay (RIA)**

Es wird exakt der Gebrauchsanweisung des RIA-Kits (Renin-Maia®-Kit, Serono Diagnostics S.A., Coinsins, Schweiz) befolgt.

Die Eichkurve umfaßt den Bereich von 0,2 bis 25,0 ng Angiotensin I pro ml. Der Basis-Angiotensin I-Gehalt des Plasmas wird von allen Meßwerten abgezogen. Die Plasma-Renin-Aktivität (PRA) wird als ng Ang I/ml x Stunde angegeben. PRA-Werte in Gegenwart der Testsubstanzen werden auf einen Ansatz ohne Inhibitor (= 100 %) bezogen und als % Restaktivität angegeben. Aus der Auftragung von % Restaktivität gegen die Konzentration (M) des Testpräparates (logarithmische Skala) wird der $IC_{50}$-Wert abgelesen.

Die erfindungsgemäßen Verbindungen der allgemeinen Formel I zeigen folgende $IC_{50}$-Werte:

| Beispiel | $IC_{50}$ (µM) |
|---|---|
| 1 | 2,0 |
| 2 | 0,62 |
| 3 | 0,9 |
| 4 | 0,38 |
| 5 | 1,0 |
| 6 | 0,4 |
| 7 | 0,26 |
| 8 | >10 |
| 9 | 6,5 |
| 10 | > 1 |
| 11 | 0,9 |
| 12 | 5,5 |
| 13 | 0,34 |
| 14 | 8 |
| 29 | 0,65 |

Renin-Hemmer bewirken an salzverarmten Tieren eine Blutdrucksenkung. Da sich menschliches Renin von dem Renin anderer Spezies unterscheidet, werden zum in-vivo-Test von Renin-Hemmern Primaten (Marmosets, Rhesus-Affen) herangezogen. Primaten-Renin und Human-Renin sind in ihrer Sequenz weitgehend homolog. Durch i.v. Injektion von Furosemid wird eine endogene Renin-Ausschüttung angeregt. Anschließend werden die Testverbindungen durch kontinuierliche Infusion verabreicht und ihre Wirkung auf Blutdruck und Herzfrequenz wird gemessen. Die Verbindungen der vorliegenden Erfindung sind hierbei in einem Dosisbereich von etwa 0,1 - 5 mg/kg i.v. wirksam, bei intraduodenaler Applikation per Gastroskop im Dosisbereich von etwa 1-50 mg/kg. Die in der vorliegenden Erfindung beschriebenen Verbindungen der allgemeinen Formel I können als Antihypertensiva, sowie zur Behandlung der Herzinsuffizienz verwendet werden.

Die Erfindung betrifft daher auch die Verwendung von Verbindungen der Formel I als Heilmittel und pharmazeutische Präparate, die diese Verbindung enthalten. Bevorzugt ist die Anwendung bei Primaten, insbesondere beim Menschen.

Pharmazeutische Präparate enthalten eine wirksame Menge des Wirkstoffs der Formel I zusammen mit einem anorganischem oder organischen pharmazeutisch verwendbaren Trägerstoff. Die Anwendung kann intranasal, intravenös, subkutan oder peroral erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier-oder Dragierverfahren hergestellt.

Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispeilsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

**Verzeichnis der verwendeten Abkürzungen:**

Boc tert.-Butoxycarbonyl
DCI Desorption Chemical Ionisation

DNP 2,4-Dinitrophenyl
EI Electron Impact
FAB Fast atom bombardment
M Molekularpeak
MeOH Methanol
MS Massenspektrum
R.T. Raumtemperatur
Schmp. Schmelzpunkt
Thi ß-2-Thienylalanin
THF Tetrahydrofuran

Die sonstigen für Aminosäuren verwendeten Abkürzungen entsprechen dem in der Peptidchemie üblichen Drei-Buchstaben-Code wie er z.B. in Eur. J. Biochem. 138, 9-37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

**Beispiel 1**

1-Cyclohexyl-6-(2-pyridyl)-2S-(N-(ethylaminocarbonyl-Phe-His)-amino)-3S-hexanol

1a) H-His(DNP)-OH

Zu einer Lösung von 0,42 g (1 mmol) Boc-His(DNP)-OH in 5 ml Dimethoxyethan tropft man bei 0-5 °C 4 ml HCl-gesättigtes Dimethoxyethan und rührt 1 Stunde bei 0-5 °C und 3 Stunden bei Raumtemperatur nach. Die Reaktionslösung wird im Vakuum eingeengt und noch zweimal mit Toloul abgeraucht.
Ausbeute: 0,5 g der Titelverbindung als Hydrochlorid Rf (Methylenchlorid/MeOH/AcOH/Wasser 70:30:1:1) = 0,16

1b) Boc-Phe-His(DNP)-OH

Zu 0,5 g (1 mmol) H-His(DNP)-OH-hydrochlorid in 12,5 ml einer 0,25 N Natriumhydrogencarbonatlösung gibt man bei Raumtemperatur 0,362 g (1 mmol) Boc-Phe-hydroxysuccinimidester, gelöst in 5 ml Ethanol und 5 ml THF. Das Reaktionsgemisch wird drei Tage bei Raumtemperatur gerührt. Dann setzt man 0,83 g Citronensäure zu, wobei die Titelverbindung ölig ausfällt. Das Produkt wird mit Methylenchlorid extrahiert, die organische Phase über Natriumsulfat getrocknet, eingeengt und der Rückstand mit wenig Essigester versetzt, die Titelverbindung durch Zusatz von Diisopropylether ausgefällt und abgesaugt.
Ausbeute: 0,47 g (83 %)
Rf (Methylenchlorid/MeOH 7:3) = 0,43
MS (FAB) = 569 (M+1)

1c) 2S-Amino-1-cyclohexyl-6-(2-pyridyl)-3S-hexanol

Zu 214 mg (0,513 mmol) 3-Boc-4S-cyclohexylmethyl-2,2-dimethyl-5-(3-(2-pyridyl)-propyl)-oxazolidin in 10 ml Dimethoxymethan tropft man im Eisbad 5 ml HCl-gesättigtes Dimethoxyethan und rührt eine Stunde bei 0 °C und 5 Stunden bei Raumtemperatur nach. Die Reaktionslösung wird im Vakuum eingeengt und noch zweimal mit Toluol abgeraucht.
Ausbeute: 179 mg der Titelverbindung als Dihydrochlorid

1d) 1-Cyclohexyl-6-(2-pyridyl)-2S-(N-(Boc-Phe-His(DNP))-amino)-3S-hexanol

1,5 g (3,18 mmol) 2S-Amino-1-cyclohexyl-6-(2-pyridyl)-3S-hexanol-dihydrochlorid (Beispiel 1c) und 1,8 g (3,18 mmol) Boc-Phe-His(DNP)-OH (Beispiel 1b) werden in 15 ml absolutem Dimethylformamid gelöst.

Dazu gibt man 0,59 g (6,36 mmol) 1-Hydroxybenzotriazol-hydrat und kühlt im Eisbad auf ca. 4 °C ab. Bei dieser Temperatur gibt man nacheinander 2,44 ml (19,1 mmol) N-Ethylmorpholin und 0,65 g (3,18 mmol) Dicyclohexylcarbodiimid zu und rührt 1 Stunde im Eisbad sowie 7 Stunden bei Raumtemperatur nach. Nach Stehen über Nacht werden erneut 0,9 g (1,59 mmol) Boc-Phe-His(DNP)-OH, 0,28 g (3,18 mmol) 1-Hydroxybenzotriazol-hydrat und 0,32 g (1,58 mmol) Dicyclohexylcarboddiimid hinzugegeben und weitere 10 Stunden bei Raumtemperatur gerührt. Man saugt vom Niederschlag ab, engt das Filtrat im Vakuum ein, löst den Rückstand in Essigester und wäscht mit gesättigter Natriumhydrogencarbonatlösung, Wasser und gesättigter Natriumchloridlösung, trocknet über Natriumsulfat, engt ein und reinigt das Rohprodukt (3,8 g) durch Mitteldruck-Säulenchromatographie an Kieselgel (Methylenchlorid/MeOH 98:2, 95:5, 9:1). Man erhält 1,8 g (68 %) der Titelverbindung.
Rf (Methylenchlorid/MeOH 9:1) = 0,46
MS (FAB) = 827 (M+1)

1e) 1-Cyclohexyl-6-(2-pyridyl)-2S-(N-(H-Phe-His(DNP))-amino)-3S-hexanol

72 mg 1-Cyclohexyl-6-(2-pyridyl)-2S-(N-(Boc-Phe-His(DNP))-amino-3S-hexanol werden bei 0-5 °C mit 2 ml eiskalter Trifluoressigsäure übergossen und zwei Stunden bei dieser Temperatur nachgerührt. Die Reaktionslösung wird im Vakuum eingeengt und zweimal mit Toluol abgeraucht.
Ausbeute: 83 mg der Titelverbindung als Bis(trifluoracetat)

1f) 1-Cyclohexyl-6-(2-pyridyl)-2S-(N-(ethylaminocarbonyl-Phe-His(DNP))-amino)-3S-hexanol

83 mg (0,087 mmol) 1-Cyclohexyl-6-(2-pyridyl)-2S-(N-(H-Phe-His(DNP))-amino)-3S-hexanol-bis-(trifluoracetat) (Beispiel 1e) werden in 2 ml absolutem Dimethylformamid gelöst. Dazu gibt man bei Raumtemperatur nacheinander 0,0365 ml (0,26 mmol) Triethylamin und 0,01 ml (0,124 mmol) Ethylisocyanat und läßt über Nacht bei Raumtemperatur stehen. Dann gibt man noch einmal 0,01 ml (0,124 mmol) Ethylisocyanat und einen Tag später erneut 0,05 ml (0,62 mmol) Ethylisocyanat zu, rührt weitere 9 Stunden bei Raumtemperatur, gibt auf Eiswasser und extrahiert mit Essigester. Die vereinigten organischen Phasen werden mit gesättigter Natriumhydrogencarbonatlösung, Wasser und gesättigter Natriumchloridlösung gewaschen, getrocknet, eingeengt und das Rohprodukt (84 mg) durch Mitteldruck-Säulenchromatographie an Kieselgel gereinigt (Methylenchlorid/MeOH 98:2, 95:5, 9:1).
Ausbeute: 48,6 mg (70 %) der Titelverbindung
Rf (Methylenchlorid/MeOH 9:1) = 0,29
MS (FAB) = 798 (M+1)

1g) 1-Cyclohexyl-6-(2-pyridyl)-2S-(N-(ethylaminocarbonyl-Phe-His)-amino)-3S-hexanol

48,6 mg (0,061 mmol) 1-Cyclohexyl-6-(2-pyridyl)-2S-(N-(ethylaminocarbonyl-Phe-His(DNP))-amino)-3S-hexanol werden in 5 ml Acetonitril mit 0,1 ml (1 mmol) Thiophenol versetzt und 5 Stunden bei Raumtemperatur gerührt. Die Reaktionslösung wird im Vakuum eingeengt und das Rohprodukt durch Mitteldruck-Säulenchromatographie an Kieselgel (Methylenchlorid/MeOH 98:2, 95:5, 9:1) gereinigt.
Ausbeute: 12,3 mg der Titelverbindung
MS (FAB) = 632 (M+1)

**Beispiel 2**

1-Cyclohexyl-6-(2-pyridyl)-2S-(N-(isopropylaminocarbonyl-Phe-Nva)-amino)-3S-hexanol

2a) Boc-Phe-Nva-OMe

15,0 g (0,056 mol) Boc-Phe-OH und 9,4 g (0,056 mol) Nva-OMe-hydrochlorid werden in 250 ml absolutem Methylenchlorid gelöst. Dazu tropft man unter Eiskühlung zunächst 38,6 ml (0,28 mol) absolutes

Triethylamin und dann 36,4 ml Propanphosphonsäureanhydrid (50 % in Methylenchlorid).

Die Reaktionslösung wird 3 Stunden bei Raumtemperatur nachgerührt und über Nacht stehengelassen. Zur Hydrolyse gibt man auf Eiswasser, rührt 2 Stunden kräftig nach, trennt die organische Phase ab und wäscht sie mit 10 %iger Citronensäurelösung, gesättigter Natriumhydrogencarbonatlösung und Wasser, trocknet über Natriumsulfat und engt ein.
Ausbeute: 20,8 g (98 %) der Titelverbindung
Rf (Methylenchlorid/MeOH 9:1) = 0,64
MS (EI) = 378 (M)

2b) Boc-Phe-Nva-OH

20,1 g (0,053 mol) Boc-Phe-Nva-OMe (Beispiel 2a) werden in 30 ml Wasser und 30 ml Dioxan suspendiert. Bei Raumtemperatur werden 2,5 g (0,106 mol) Lithiumhydroxid eingetragen und 3 Stunden bei Raumtemperatur nachgerührt. Die Reaktionslösung wird mit 10 %iger Natriumhydrogensulfatlösung angesäuert und das Produkt abgesaugt und mit Diisopropylether verrührt.
Ausbeute: 19,1 g (98 %)
Rf (Methylenchlorid/MeOH 9:1) = 0,18
MS (FAB) = 365 (M+1)

2c) 1-Cyclohexyl-6-(2-pyridyl)-2S-(N-(Boc-Phe-Nva)-amino)-3S-hexanol

Aus 1,7 g (3,36 mmol) 2S-Amino-1-cyclohexyl-6-(2-pyridyl)-3S-hexanoldihydrochlorid (Beispiel 1c) und 1,22 g (3,36 mmol) Boc-Phe-Nva-OH (Beispiel 2b) erhält man analog dem in Beispiel 1d) angegebenen Verfahren 0,8 g der Titelverbindung.
Rf (Methylenchlorid/MeOH 9:1) = 0,53
MS (FAB) = 623 (M+1)

2d) 1-Cyclohexyl-6-(2-pyridyl)-2S-(N-(H-Phe-Nva)-amino)-3S-hexanol

Aus 97 mg 1-Cyclohexyl-6-(2-pyridyl)-2S-(N-(Boc-Phe-Nva)-amino)-3S-hexanol (Beispiel 2c) erhält man analog dem in Beispiel 1a) angegebenen Verfahren 92 mg der Titelverbindung als Dihydrochlorid.

2e) 1-Cyclohexyl-6-(2-pyridyl)-2S-(N-(isopropylaminocarbonyl-Phe-Nva)-amino)-3S-hexanol

92 mg (0,155 mmol) 1-Cyclohexyl-6-(2-pyridyl)-2S-N-(H-Phe-Nva)-amino)-3S-hexanol-dihydrochlorid (Beispiel 2d) werden in 5 ml absolutem Dimethylformamid gelöst. Bei Raumtemperatur werden nacheinander 0,104 ml (0,73 mmol) Triethylamin und 0,019 ml (0,19 mmol) Isopropylisocyanat zugegeben. Man läßt übers Wochenende bei Raumtemperatur stehen, engt im Vakuum ein, nimmt den Rückstand in Essigester auf, wäscht mit gesättigter Natriumhydrogencarbonatlösung, Wasser und gesättigter Natriumchloridlösung, trocknet über Natriumsulfat, engt ein und reinigt das Rohprodukt (98 mg) durch Mitteldruck-Säulenchromatographie an Kieselgel (Methylenchlorid/MeOH 95:5).
Ausbeute: 13,3 mg der Titelverbindung
Rf (Methylenchlorid/MeOH 9:1) = 0,39
MS (FAB) = 608 (M+1)

**Beispiel 3**

1-Cyclohexyl-6-(2-pyridyl)-2S-(N-(2,2-dimethylpropylaminocarbonyl-Phe-Nva)-amino)-3S-hexanol

Zu 126 mg (0,32 mmol) Di(benzotriazolyl). carbonat in 10 ml absolutem Methylenchlorid und 0,026 ml (0,32 mmol) Pyridin tropft man bei -75 bis -70 °C eine Lösung von 84 mg (0,16 mmol) 1-Cyclohexyl-6-(2-pyridyl)-2S-(N-(H-Phe-Nva)-amino)- 3S-hexanol (Beispiel 2d, freigesetzt aus dem Hydrochlorid durch Behandlung mit Natriumbicarbonat) in 10 ml absolutem Methylenchlorid und rührt die Lösung 1 Stunde bei dieser Temperatur nach. Dann läßt man auf Raumtemperatur kommen, rührt 3 Stunden nach und kühlt erneut auf -75 °C ab. Man gibt nun 0,0955 mol (0,8 mmol) 2,2-Dimethyl-1-propylamin zu, rührt 2 Stunden bei -75 °C und läßt über Nacht bei Raumtemperatur stehen. Die Reaktionslösung wird im Vakuum eingeengt, der Rückstand in Essigester aufgenommen, mit gesättigter Natriumbicarbonatlösung, Wasser und gesättigter Natriumchloridlösung gewaschen, über Natriumsulfat getrocknet, eingeengt und das Rohprodukt (144 mg) durch Mitteldruck-Säulenchromatographie an Kieselgel (Methylenchlorid/MeOH 100:0, 98:2, 95:5) gereinigt.
Ausbeute: 39 mg (39 %) der Titelverbindung
Rf (Methylenchlorid/MeOH 9:1) = 0,32
MS (FAB) = 636 (M+1)

Unter Verwendung geeigneter Ausgangsmaterialien und unter Anwendung der in den Beispielen 1 bis 3 beschriebenen Verfahren wurden die in den folgenden Beispielen 4 bis 14 beschriebenen Verbindungen erhalten:

**Beispiel 4**

1-Cyclohexyl-6-(2-pyridyl)-2S-(N-(ethylaminocarbonyl-Phe-Nva)-amino)-3S-hexanol

Rf (Methylenchlorid/MeOH 9:1) = 0,42
MS (FAB) = 594 (M+1)

**Beispiel 5**

1-Cyclohexyl-6-(2-pyridyl)-2S-(N-(tert.butylaminocarbonyl)-Phe-His)-amino)-3S-hexanol

Rf (Methylenchlorid/MeOH 9:1) = 0,25
MS (FAB) = 660 (M+1)

**Beispiel 6**

1-Cyclohexyl-6-(2-pyridyl)-2S-(N-(tert.butylaminocarbonyl-Phe-Nva)-amino)-3S-hexanol

Rf (Methylenchlorid/MeOH 9:1) = 0,52
MS (FAB) = 622 (M+1)

**Beispiel 7**

1-Cyclohexyl-6-(2-pyridyl)-2S-(N-(tert.butylaminocarbonyl-Thi-Nva)-amino)-3S-hexanol

Rf (Methylenchlorid/MeOH 9:1) = 0,39
MS (FAB) = 628 (M+1)

**Beispiel 8**

1-Cyclohexyl-6-(2-pyridyl)-2S-(N-(tert.butylaminocarbonyl)-Tyr(OMe)-Nva)-amino)-3S-hexanol

Rf (Methylenchlorid/MeOH 9:1) = 0,38
MS (FAB) = 652 (M+1)

**Beispiel 9**

1-Cyclohexyl-6-(2-pyridyl)-2S-(N-(tert.butylaminocarbonyl-Phe-Nva)-amino)-3R-hexanol

Rf (Toluol/EtOH 9:1) = 0,33
MS (FAB) = 622 (M+1)

**Beispiel 10**

1-Cyclohexyl-6-(2-pyridyl)-2S-(N-(tert.butylaminocarbonyl-Phe-His)-amino)-3R-hexanol

Rf (Methylenchlorid/MeOH 9:1) = 0,32
MS (FAB) = 660 (M+1)

**Beispiel 11**

1-Cyclohexyl-6-(2-pyridyl)-2S-(N-(ethylaminocarbonyl-Phe-His)-amino)-3R-hexanol

MS (FAB) = 632 (M+1)

**Beispiel 12**

1-Cyclohexyl-6-(2-pyridyl)-2S-(N-(isopropylaminocarbonyl-Phe-Nva)-amino)-3R-hexanol

Rf (Methylenchlorid/MeOH 9:1) = 0,46
MS (FAB) = 608 (M+1)

**Beispiel 13**

1-Cyclohexyl-6-(2-pyridyl)-2S-(N-(2-amino-2-methylpropylaminocarbonyl-Phe-Nva)-amino)-3S-hexanol

Rf (Methylenchlorid/MeOH 7:3) = 0,09
MS (FAB) = 637 (M+1)

**Beispiel 14**

1-Cyclohexyl-6-(2-pyridyl)-2S-(N-tert.butylaminocarbonyl-Phe-Asn)-amino)-3S-hexanol

Rf (Methylenchlorid/MeOH 9:1) = 0,24
MS (FAB) = 637 (M+1)

Die folgenden Verbindungen 15-28 wurden analog dem in EP-A-255 082 beschriebenen "herkömmlichen" Verfahren hergestellt.

**Beispiel 15**

1-Cyclohexyl-6-(2-pyridyl)-2S-(N-(ethylaminocarbonyl-Phe-Nva)-amino)-3S-hexanol

15a) N-(Ethylaminocarbonyl)-Phe-OH

3,37 g (10 mmol) Phe-p-toluolsulfonat werden in 10 ml absolutem Tetrahydrofuran gelöst, bei Raumtemperatur 2,4 ml (12 mmol) frisch destilliertes Hexamethyldisilazan hinzugegeben und zwei Stunden nachgerührt. Zu der Suspension gibt man 0,97 ml (12 mmol) Ethylisocyanat und läßt über Nacht bei

Raumtemperatur stehen. Man saugt vom Niederschlag ab, kühlt das Filtrat im Eisbad ab, filtriert erneut, engt ein und verrührt den Rückstand mit Wasser. Der Niederschlag wird abgesaugt und über Phosphorpentoxid getrocknet.
Ausbeute: 1,7 g (72 %)
Rf (Methylenchlorid/MeOH/Wasser/Eisessig 70:30:1:1) = 0,68
MS (DCI) = 237 (M+1)

15b) N-(Ethylcarbonylamino)-Phe-Nva-OMe

Zu 1,2 g (5,08 mmol) N-(Ethylaminocarbonyl)-Phe-OH aus Beispiel 15a) und 0,85 g (5,08 mmol) L-Nva-OMe-hydrochlorid in 30 ml absolutem Methylenchlorid gibt man unter Eiskühlung nacheinander 3,51 ml (25,4 mmol) absolutes Triethylamin und 3,30 ml Propanphosphonsäureanhydrid (50 % in Methylenchlorid), rührt 6 Stunden bei Raumtemperatur nach und läßt über Nacht stehen. Das Reaktionsgemisch wird zur Hydrolyse auf Eiswasser gegeben, die organische Phase abgetrennt und je dreimal mit jeweils 100 ml 10 %iger Citronensäurelösung, gesättigter Natriumhydrogencarbonatlösung und Wasser gewaschen, über Natriumsulfat getrocknet und eingeengt. Der Rückstand wird in wenig kaltem Diisopropylether verrührt, abgesaugt und im Vakuum getrocknet.
Ausbeute: 1,5 g (84 %)
Rf (Methylenchlorid/MeOH 9:1) = 0,49

15c) N-(Ethylaminocarbonyl)-Phe-Nva-OH

Zu 1,5 g (4,29 mmol) N-(Ethylaminocarbonyl)-Phe-Nva-OMe aus Beispiel 15b) in 8 ml Wasser und 8 ml Dioxan gibt man bei Raumtemperatur 0,2 g Lithiumhydroxid und rührt zwei Stunden nach. Die Reaktionslösung wird mit 10 %iger Natriumhydrogensulfatlösung sauer gestellt, der Niederschlag abgesaugt, mit Diisopropylether verrührt und getrocknet.
Ausbeute: 1,3 g
Rf (Toluol/Ethanol 8:2) = 0,02
MS (DCI) = 336 (M+1)

15d) 2S-Amino-1-cyclohexyl-6-(2-pyridyl)-3S-hexanol-dihydrochlorid

Zu 214 mg (0,513 mmol) 3-Boc-4S-cyclohexylmethyl-2,2-dimethyl-5-(3-(2-pyridyl)-propyl)-oxazolidin in 10 ml Dimethoxymethan tropft man im Eisbad 5 ml HCl-gesättigtes Dimethoxymethan und rührt eine Stunde bei 0 °C sowie 5 Stunden bei Raumtemperatur nach. Die Reaktionslösung wird im Vakuum eingeengt und noch zweimal mit Toluol abgeraucht.
Ausbeute: 179 mg

15e) 1-Cyclohexyl-6-(2-pyridyl)-2S-(N-(ethylaminocarbonyl-Phe-Nva)-amino)-3S-hexanol

160,5 mg (0,513 mmol) 2S-Amino-1-cyclohexyl-6-(2-pyridyl)-3S-hexanol-dihydrochlorid aus Beispiel 15d) und 172 mg (0,513 mmol) N-(Ethylamino-carbonyl)-Phe-Nva-OH aus Beispiel 15c) werden in 2 ml absolutem Dimethylformamid gelöst. Bei Raumtemperatur setzt man 273 mg (1,03 mmol) 1-Hydroxybenzotriazol zu und kühlt auf 0 °C ab. Bei dieser Temperatur gibt man zuerst 0,65 ml (5,13 mmol) N-Ethylmorpholin zu, danach 106 mg (0,513 mmol) Dicyclohexylcarbodiimid und rührt 5 Stunden bei Raumtemperatur nach. Dann gibt man weitere 100 mg (0,298 mmol) N-(Ethylaminocarbonyl)-Phe-Nva-OH zu und läßt über Nacht stehen. Man saugt vom Dicyclohexylharnstoff ab, gibt das Filtrat auf Wasser und extrahiert mehrmals mit Essigester. Die vereinigten organischen Phasen werden mit gesättigter Natriumhydrogencarbonatlösung, Wasser und Natriumchloridlösung gewaschen, über Natriumsulfat getrocknet, eingeengt und das Rohprodukt (335 mg) durch Mitteldruck-Säulenchromatographie an Kieselgel (Laufmittel Methylenchlorid/Methanol 100:0, 98:2, 95:5, 9:1) gereinigt. Man erhält 98 mg (32 %) der Titelverbindung.
Rf (Methylenchlorid/MeOH 9:1) = 0,41
MS (FAB) = 594 (M+1)

**Beispiel 16**

1-Cyclohexyl-6-(2-pyridyl)-2S-(N-(tert.butylaminocarbonyl-Phe-His)-amino)-3S-hexanol

16a) N-(Tert.butylaminocarbonyl)-Phe-OH

Die Titelverbindung wird analog Beispiel 15a) aus 3,37 g L-Phenylalanin-p-toluolsulfonat und Tert.butylisocyanat erhalten.
Ausbeute: 1,6 g (60 %)
Rf (Methylenchlorid/MeOH/Wasser/Eisessig 70:30:1:1) = 0,76
MS (DCI) = 265 (M+1)

16b) 2S-(N-(Boc-His(DNP))-amino)-1-cyclohexyl-6-(2-pyridyl)-3S-hexanol

Zu 1,9 g Boc-His(DNP)-OH, 0,36 ml Pyridin und 0,62 ml N-Ethylpiperidin in 50 ml Methylenchlorid werden bei -5 °C 0,55 ml Pivaloylchlorid getropft. Nach 10 Minuten bei -5 °C rührt man 10 Minuten bei +10 °C. Nach erneutem Abkühlen auf -5 °C gibt man 1,3 g 2S-Amino-1-cyclohexyl-6-(2-pyridyl)-3S-hexanol (freigesetzt aus dem Dihydrochlorid aus Beispiel 15d) mit Natriumcarbonat) in 20 ml Methylenchlorid zu. Nach einer Stunde bei -5 °C läßt man 16 Stunden bei Raumtemperatur stehen. Man versetzt mit 50 ml gesättigter Natriumcarbonatlösung und extrahiert dreimal mit Essigester. Die vereinigten organischen Extrakte werden über Natriumsulfat getrocknet und eingeengt und durch Chromatographie an Kieselgel (EE/MeOH 10:1) gereinigt.
Rf (EE/MeOH 10:1) = 0,25
MS (FAB) = 680 (M+1)

16c) 2S-(N-His(DNP)-amino)-1-cyclohexyl-6-(2-pyridyl)-3S-hexanol-dihydrochlorid

Zu 170 mg 2S-(N-(Boc-His(DNP))-amino)-1-cyclohexyl-6-(2-pyridyl)-3S-hexanol aus Beispiel 16b) in 10 ml Dimethoxyethan tropft man bei 0-5 °C 26 ml HCl-gesättigtes Dimethoxyethan und rührt eine Stunde im Eisbad und 4 Stunden bei Raumtemperatur nach. Die Reaktionslösung wird eingeengt und noch zweimal mit Toluol versetzt und eingeengt.
Ausbeute: 167 mg der Titelverbindung

16d) 1-Cyclohexyl-6-(2-pyridyl)-2S-(N-(tert.butylaminocarbonyl-Phe-His(DNP))-amino)-3S-hexanol

Die Titelverbindung wird erhalten ausgehend von 167 mg (0,256 mmol) 2S-(N-His(DNP)-amino)-1-cyclohexyl-6-(2-pyridyl)-3S-hexanol-dihydrochlorid (Beispiel 16c) und 68 mg (0,256 mmol) N-Tert.butylaminocarbonyl-Phe-OH (Beispiel 16a) analog dem im Beispiel 15e) beschriebenen Verfahren.
Ausbeute: 83,7 mg (40 %) der Titelverbindung
Rf (Methylenchlorid/MeOH 9:1) = 0,44
MS (FAB) = 826 (M+1)

16e) 1-Cyclohexyl-6-(2-pyridyl)-2S-(N-(tert.butylaminocarbonyl-Phe-His)-amino)-3S-hexanol

83 mg (0,1 mmol) 1-Cyclohexyl-6-(2-pyridyl)-2S-(N-(tert.butylamino-carbonyl-Phe-His(DNP))-amino)-3S-hexanol (Beispiel 16d) werden in 5 ml Acetonitril gelöst und mit 0,1 ml (1 mmol) Thiophenol 6 Stunden bei Raumtemperatur gerührt. Die Reaktionslösung wird im Vakuum eingeengt und der Rückstand durch Mitteldruck-Säulenchromatographie an Kieselgel (Methylenchlorid/MeOH 100:0, 98:2, 95:5, 9:1) gereinigt.
Ausbeute: 51 mg (77 %) der Titelverbindung
Rf (Methylenchlorid/MeOH 9:1) = 0,26
MS (FAB) = 660 (M+1)

**Beispiel 17**

1-Cyclohexyl-6-(2-pyridyl)-2S-(N-(tert.butylaminocarbonyl-Phe-Nva)-amino)-3S-hexanol

17a) N-Tert.butylaminocarbonyl-Phe-Nva-OMe

Aus 264 mg (1 mmol) N-Tert.butylaminocarbonyl-Phe-OH (Beispiel 16a) und 167 mg (1 mmol) Nva-OMe-Hydrochlorid wird nach dem in Beispiel 15b) beschriebenen Verfahren die Titelverbindung erhalten.
Ausbeute: 0,35 g (93 %)
Rf (Methylenchlorid/MeOH 9:1) = 0,69
MS (DCI) = 378 (M+1)

17b) N-Tert.butylaminocarbonyl-Phe-Nva-OH

Aus 100 mg (0,265 mmol) N-Tert.butylaminocarbonyl-Phe-Nva-OMe (Beispiel 17a) wird nach dem im Beispiel 15c) beschriebenen Verfahren die Titelverbindung erhalten.
Ausbeute: 90 mg (93 %)
MS (DCI) = 364 (M+1)

17c) 1-Cyclohexyl-6-(2-pyridyl)-2S-(N-(tert.butylaminocarbonyl)-Phe-Nva)-amino)-3S-hexanol

Aus 166 mg (0,529 mmol) 2S-Amino-1-cyclohexyl-6-(2-pyridyl)-3S-hexanol-dihydrochlorid (Beispiel 15d) und 192 mg (0,529 mmol) N-Tert.butylaminocarbonyl-Phe-Nva-OH wird nach dem in Beispiel 15e) beschriebenen Verfahren die Titelverbindung erhalten.
Ausbeute: 121 mg (37 %)
Rf (Methylenchlorid/MeOH 9:1) = 0,52
MS (FAB) = 622 (M+1)

**Beispiel 18**

1-Cyclohexyl-6-(2-pyridyl)-2S-(N-tert.butylaminocarbonyl-Thi-Nva)-amino)-3S-hexanol

18a) N-Tert.butylaminocarbonyl-Thi-OH

Aus 1,5 g (4,82 mmol) L-Thienylalanin-p-toluolsulfonat und 0,68 ml (5,78 mmol) Tert.butylisocyanat wird nach dem in Beispiel 15a) beschriebenen Verfahren die Titelverbindung erhalten.
Ausbeute: 0,7 g
Rf (Methylenchlorid/MeOH/AcOH/Wasser 70:30:1:1) = 0,9
MS (FAB) = 271 (M+1)

18b) N-Tert.butylaminocarbonyl-Thi-Nva-OMe

Aus 0,7 g (2,6 mmol) N-tert.butylaminocarbonyl-Thi-OH (Beispiel 18a) und 0,44 g (2,6 mmol) Nva-OMe-hydrochlorid wird nach dem in Beispiel 15b) beschriebenen Verfahren die Titelverbindung erhalten.
Ausbeute: 0,8 g (80 %)
MS (FAB) = 384 (M+1)

18c) N-Tert.butylaminocarbonyl-Thi-Nva-OH

Aus 0,8 g (2,1 mmol) N-Tert.butylaminocarbonyl-Thi-Nva-OMe (Beispiel 18b) wird nach dem in Beispiel 15c) beschriebenen Verfahren die Titelverbindung erhalten.
Ausbeute: 0,53 g (69 %)
Rf (Methylenchlorid/MeOH 9:1) = 0,08
MS (FAB) = 369 (M+1)

18d) 1-Cyclohexyl-6-(2-pyridyl)-2S-(N-(tert.butylaminocarbonyl-Thi-Nva)-amino)-3S-hexanol

Aus 92 mg (0,25 mmol) N-Tert.butylaminocarbonyl-Thi-Nva-OH (Beispiel 18c) und 87 mg (0,25 mmol) 2S-Amino-1-cyclohexyl-6-(2-pyridyl)-3S-hexanol-dihydrochlorid (Beispiel 15d) wird analog dem in Beispiel 15e) beschriebenen Verfahren die Titelverbindung erhalten.
Ausbeute: 59,6 mg (38 %)
Rf (Methylenchlorid/MeOH 9:1) = 0,39
MS (FAB) = 628 (M+1)

**Beispiel 19**

1-Cyclohexyl-6-(2-pyridyl)-2S-(N-tert.butylaminocarbonyl-Tyr(OMe)-Nva)-amino)-3S-hexanol

19a) N-Tert.butylaminocarbonyl-Tyr(OMe)-OH

Aus 3,0 g (8,16 mmol) L-(O-Methyltyrosin)-p-toluolsulfonat und 1,15 ml (9,79 mmol) Tert.butylisocyanat wird analog dem in Beispiel 15a) beschriebenen Verfahren die Titelverbindung hergestellt.
Ausbeute: 1,7 g (70 %)
MS (FAB) = 295 (M+1)

19b) N-Tert.butylaminocarbonyl-Tyr(OMe)-Nva-OMe

Aus 0,7 g (2,38 mmol) N-Tert.butylaminocarbonyl-Tyr(OMe)-OH und 0,4 g (2,38 mmol) Nva-OMe-hydrochlorid wird analog dem in Beispiel 15b) beschriebenen Verfahren die Titelverbinding hergestellt.
Ausbeute: 0,8 g (82 %)
Rf (Methylenchlorid/MeOH 9:1) = 0,66
MS (FAB) = 408 (M+1)

19c) N-Tert.butylaminocarbonyl-Tyr(OMe)-Nva-OH

Aus 0,8 g (1,96 mmol) N-Tert.butylaminocarbonyl-Tyr(OMe)-Nva-OMe (Beispiel 19b) wird analog dem in Beispiel 15c) beschriebenen Verfahren die Titelverbindung erhalten.
Ausbeute: 0,7 g (91 %)
Rf (Methylenchlorid/MeOH 9:1) = 0,13
MS (FAB) = 394 (M+1)

19d) 1-Cyclohexyl-6-(2-pyridyl)-2S-(N-(tert.butylaminocarbonyl-Tyr(OMe)-Nva)-amino)-3S-hexanol

Aus 110 mg (0,28 mmol) N-Tert.butylaminocarbonyl-Tyr(OMe)-Nva-OH (Beispiel 19c) und 98 mg (0,28 mmol) 2S-Amino-1- cyclohexyl-6-(2-pyridyl)-3S-hexanol (Beispiel 15d) wird analog dem im Beispiel 15e) beschriebenen Verfahren die Titelverbindung erhalten.
Ausbeute: 35,2 mg (19 %)
Rf (Methylenchlorid/MeOH 9:1) = 0,38
MS (FAB) = 652 (M+1)

Unter Verwendung geeigneter Ausgangsmaterialien und unter Anwendung der in den Beispielen 15 bis

19 beschriebenen Verfahren wurden die in den folgenden Beispielen 20 bis 29 beschriebenen Verbindungen erhalten:

**Beispiel 20**

1-Cyclohexyl-6-(2-pyridyl)-2S-(N-(tert.butylaminocarbonyl-Phe-Nva)-amino)-3R-hexanol

Rf (Toluol/Ethanol 9:1) = 0,32
MS (FAB) = 622 (M+1)

**Beispiel 21**

1-Cyclohexyl-6-(2-pyridyl)-2S-(N-(tert.butylaminocarbonyl-Phe-His)-amino)-3R-hexanol

Rf (Methylenchlorid/MeOH 9:1) = 0,32
MS (FAB) = 660 (M+1)

**Beispiel 22**

1-Cyclohexyl-6-(2-pyridyl)-2S-(N-(ethylaminocarbonyl-Phe-His)-amino)-3R-hexanol

MS (FAB) = 632 (M+1)

**Beispiel 23**

1-Cyclohexyl-6-(2-pyridyl)-2S-(N-(isopropylaminocarbonyl-Phe-Nva)-amino)-3R-hexanol

Rf (Methylenchlorid/Methanol 9:1) = 0,46
MS (FAB) = 608 (M+1)

**Beispiel 24**

1-Cyclohexyl-6-(2-pyridyl)-2S-(N-(2-amino-2-methylpropylaminocarbonyl-Phe-Nva)-amino)-3S-hexanol

Rf (Methylenchlorid/MeOH 7:3) = 0,09
MS (FAB) = 637 (M+1)

**Beispiel 25**

1-Cyclohexyl-6-(2-pyridyl)-2S-(N-(tert.butylaminocarbonyl-Phe-Asn)-amino)-3S-hexanol

Rf (Methylenchlorid/MethOH 9:1) = 0,24
MS (FAB) = 637 (M+1)

**Beispiel 26**

1-Cyclohexyl-6-(2-pyridyl)-2S-(N-(ethylaminocarbonyl-Phe-His)-amino)-3S-hexanol

MS (FAB) = 632 (M+1)

**Beispiel 27**

1-Cyclohexyl-6-(2-pyridyl)-2S-(N-(isopropylaminocarbonyl-Phe-Nva)-amino)-3S-hexanol

Rf (Methylenchlorid/MeOH 9:1) = 0,39
MS (FAB) = 608 (M+1)

**Beispiel 28**

1-Cyclohexyl-6-(2-pyridyl)-2S-(N-(2,2-dimethylpropylaminocarbonyl-Phe-Nva)-amino)-3S-hexanol

Rf (Methylenchlorid/MeOH 9:1) = 0,51
MS (FAB) = 636 (M+1)

**Beispiel 29**

1-Cyclohexyl-6-(2-pyridyl)-2S-[N-[(1-carboxy-1-methylethylaminocarbonyl)-Phe-Nva]-amino]-3S-hexanol

Rf (Methylenchlorid/MeOH 9:1) = 0,10
MS (FAB) = 652 (M+1)

## Ansprüche

1. Verbindung der Formel I

$$R^1 - A - B - HN - \underset{}{\overset{R^2}{\overset{|}{CH}}} - \overset{OH}{\overset{|}{CH}} - \overset{R^3}{\overset{|}{CH}} - R^4 \qquad (I)$$

in welcher
$R^1$ einen Rest der Formel II bedeutet
$R^a$ - NH - CO - (II)
worin
$R^a$ Wasserstoff, $(C_1\text{-}C_{10})$-Alkyl, das gegebenenfalls ein-oder zweifach ungesättigt ist und das gegebenenfalls durch bis zu 3 gleiche oder verschiedene Reste aus der Reihe Hydroxy, $(C_1\text{-}C_7)$-Alkoxy, $(C_1\text{-}C_7)$-Alkanoyloxy, Carboxy, $(C_1\text{-}C_7)$-Alkoxycarbonyl, Cl, Br, Amino, $(C_1\text{-}C_7)$-Alkylamino, Di-$(C_1\text{-}C_7)$-alkylamino, $(C_1\text{-}C_5)$-Alkoxycarbonylamino, $(C_7\text{-}C_{15})$-Aralkoxycarbonylamino und 9-Fluorenylmethyloxycarbonylamino substituiert ist,
$(C_3\text{-}C_8)$-Cycloalkyl, $(C_3\text{-}C_8)$-Cycloalkyl-$(C_1\text{-}C_6)$-alkyl, $(C_6\text{-}C_{14})$-Aryl, das gegebenenfalls durch einen oder zwei gleiche oder verschiedene Reste aus der Reihe F, Cl, Br, J, Hydroxy, $(C_1\text{-}C_7)$-Alkoxy, $(C_1\text{-}C_7)$-Alkyl, $(C_1\text{-}C_7)$-Alkoxycarbonyl, Amino, gegebenenfalls mit bis zu 2 Halogen substituiertes Anilino und Trifluormethyl substituiert ist,
$(C_6\text{-}C_{14})$-Aryl-$(C_1\text{-}C_6)$-alkyl, worin der Arylteil gegebenenenfalls durch einen oder zwei gleiche oder verschiedene Reste aus der Reihe F, Cl, Br, J, Hydroxy, $(C_1\text{-}C_7)$-Alkoxy, $(C_1\text{-}C_7)$-Alkyl, $(C_1\text{-}C_7)$-Alkoxycarbonyl, Amino, $(C_1\text{-}C_7)$-Alkylamino, Di-$(C_1\text{-}C_7)$-alkylamino, Carboxy, Carboxymethoxy, Amino-$(C_1\text{-}C_7)$-alkyl, $(C_1\text{-}C_7)$-Alkylamino-$(C_1\text{-}C_7)$-alkyl, Di-$(C_1\text{-}C_7)$-alkylamino-$(C_1\text{-}C_7)$-alkyl, $(C_1\text{-}C_7)$-Alkoxycarbonylmethoxy, Carbamoyl, Sulfamoyl, $(C_1\text{-}C_7)$-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, $(C_1\text{-}C_7)$-Alkoxy, $(C_1\text{-}C_7)$-Alkyl, $(C_1\text{-}C_7)$-Alkoxycarbonyl, Amino oder Trifluormethyl substituiert ist,
A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin und (E)-Dehydrophenylalanin bedeutet,
B einen N-terminal mit A und C-terminal mit

$$\begin{array}{ccccccccc} & & R^2 & & OH & & R^3 & & \\ & & | & & | & & | & & \\ NH & - & CH & - & CH & - & CH & - & R^4 \end{array}$$

verknüpften Rest einer Aminosäure, der wie unter A definiert ist, bedeutet,
$R^2$ Wasserstoff, $(C_1\text{-}C_{10})$-Alkyl, $(C_4\text{-}C_7)$-Cycloalkyl, $(C_4\text{-}C_7)$-Cycloalkyl-$(C_1\text{-}C_4)$-alkyl, $(C_6\text{-}C_{14})$-Aryl oder $(C_6\text{-}C_{14})$-Aryl-$(C_1\text{-}C_4)$-alkyl bedeutet und
$R^3$ Wasserstoff, $(C_1\text{-}C_{10})$-Alkyl, $(C_6\text{-}C_{14})$-Aryl oder $(C_6\text{-}C_{14})$-Aryl-$(C_1\text{-}C_4)$-alkyl bedeutet,
$R^4$ einen Rest der Formel III bedeutet
$-(CH_2)_p-X-(CH_2)_q-R^5$ (III)
worin X wahlweise abwesend sein kann oder für -O-, -S-, $-CF_2-$, -CO- oder $-CHR^6-$ steht,
p und q unabhängig voneinander 0, 1, 2, 3 oder 4 bedeuten,
$R^6$ Wasserstoff, $(C_1\text{-}C_7)$-Alkyl, $(C_1\text{-}C_5)$-Alkoxy, $(C_1\text{-}C_5)$-Alkylthio, $(C_1\text{-}C_5)$-Alkylamino-, -OH, $-N_3$, -F, -Cl, -Br oder -I bedeutet, und
$R^5$ Wasserstoff, -OH, $-NH_2$ oder Heteroaryl, das auch teilweise oder vollständig hydriert sein kann, bedeutet sowie deren physiologisch verträgliche Salze.

2. Verbindung der Formel I gemäß Anspruch 1, dadurch gekennzeichnet, daß
$R^1$ einen Rest der Formel II bedeutet, worin
$R^a$ Wasserstoff, $(C_1\text{-}C_{10})$-Alkyl, $(C_1\text{-}C_4)$-Alkoxy-$(C_1\text{-}C_{10})$-alkyl, Carboxy-$(C_1\text{-}C_{10})$-alkyl, $(C_1\text{-}C_4)$-Alkoxycarbonyl-$(C_1\text{-}C_{10})$-alkyl, Amino-$(C_1\text{-}C_{10})$-alkyl, Diamino-$(C_1\text{-}C_{10})$-alkyl, $(C_1\text{-}C_4)$-Alkylamino-$(C_1\text{-}C_{10})$-alkyl, Di$(C_1\text{-}C_4)$alkylamino-$(C_1\text{-}C_{10})$-alkyl, N,N′-Di-$(C_1\text{-}C_4)$-alkyldiamino-$(C_1\text{-}C_{10})$-alkyl, N,N,N′,N′-Tetra$(C_1\text{-}C_4)$-alkyldiamino-$(C_1\text{-}C_{10})$-alkyl, $(C_1\text{-}C_4)$-Alkoxycarbonylamino-$(C_1\text{-}C_{10})$-alkyl, $(C_7\text{-}C_{15})$-Aralkoxycarbonylamino-$(C_1\text{-}C_{10})$-alkyl, Cyclopentyl, Cyclohexyl, Cycloheptyl, Cyclopentyl-$(C_1\text{-}C_6)$-alkyl, Cyclohexyl-$(C_1\text{-}C_6)$-alkyl, Phenyl, das gegebenenfalls durch einen oder zwei gleiche oder verschiedene Reste aus der Reihe F, Cl, Br, J, Hydroxy, $(C_1\text{-}C_4)$-Alkoxy, $(C_1\text{-}C_4)$-Alkyl, $(C_1\text{-}C_4)$-Alkoxycarbonyl, Amino oder Trifluormethyl substituiert ist, $(C_6\text{-}C_{10})$-Aryl-$(C_1\text{-}C_6)$-alkyl, worin der Arylteil wie oben Phenyl substituiert sein kann, bedeutet oder für den Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, $(C_1\text{-}C_7)$-Alkoxy, $(C_1\text{-}C_7)$-Alkyl, $(C_1\text{-}C_7)$-Alkoxycarbonyl, Amino oder Trifluormethyl substituiert ist,
A einen N-terminal mit $R^1$ und C-terminal mit B verknüpften Rest einer Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin oder (E)-Dehydrophenylalanin bedeutet,
B einen N-terminal mit A und C-terminal mit

$$\begin{array}{cccccccccc} & & & R^2 & & OH & & R^3 & & \\ & & & | & & | & & | & & \\ - & NH & - & CH & - & CH & - & CH & - & R^4 \end{array}$$

verknüpften Rest einer Aminosäure aus der Reihe
Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]-thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2-Thienyl)-serin, (Z)-Dehydrophenylalanin und (E)-Dehydrophenylalanin, bedeutet,
$R^2$ Wasserstoff, $(C_1$-$C_{10})$-Alkyl, $(C_4$-$C_7)$-Cycloalkyl, $(C_4$-$C_7)$-Cycloalkyl-$(C_1$-$C_4)$-alkyl, $(C_6$-$C_{14})$-Aryl oder $(C_6$-$C_{14})$-alkyl bedeutet,
$R^3$ Wasserstoff, $(C_1$-$C_{10})$-Alkyl, $(C_6$-$C_{14})$-Aryl oder $(C_6$-$C_{14})$-Aryl-$(C_1$-$C_4)$-alkyl bedeutet,
$R^4$ einen Rest der Formel III bedeutet, worin
X wahlweise abwesend sein kann oder für -O-, -S-, $-CF_2-$, -CO- oder $-CHR^6-$ steht,
p und q unabhängig voneinander 0, 1, 2, 3 oder 4 bedeuten,
$R^6$ Wasserstoff, $(C_1$-$C_7)$-Alkyl, $(C_1$-$C_5)$-Alkoxy, $(C_1$-$C_5)$-Alkylthio, $(C_1$-$C_5)$-Alkylamino, -OH, $-N_3$, -F, -Cl, -Br oder -I bedeutet, und
$R^5$ Wasserstoff, -OH, $-NH_2$ oder Heteroaryl, das auch teilweise oder vollständig hydriert sein kann, bedeutet sowie deren physiologisch verträgliche Salze.

3. Verbindung der Formel I gemäß einem oder mehreren der Ansprüche 1-2, dadurch gekennzeichnet, daß
$R^1$ einen Rest der Formel II bedeutet, worin
$R^a$ Wasserstoff, $(C_1$-$C_6)$-Alkyl, Carboxy-$(C_1$-$C_6)$-alkyl, Amino-$(C_1$-$C_6)$-alkyl, Diamino-$(C_1$-$C_6)$-alkyl, $(C_1$-$C_4)$-Alkylamino-$(C_1$-$C_6)$-alkyl, Di-$(C_1$-$C_4)$-alkylamino-$(C_1$-$C_6)$-alkyl, N,N′-Di-$(C_1$-$C_4)$-alkyldiamino-$(C_1$-$C_6)$-alkyl, N,N,N′,N′-Tetra-$(C_1$-$C_4)$-alkyldiamino-$(C_1$-$C_6)$-alkyl, $(C_1$-$C_4)$-Alkoxycarbonylamino-$(C_1$-$C_6)$-alkyl oder Phenyl, das gegebenenfalls durch einen oder zwei gleiche oder verschiedene Reste aus der Reihe F, Cl, Hydroxy oder Amino substituiert ist, bedeutet,
A einen N-terminal mit $R^1$ und C-terminal mit B verknüpften Rest einer Aminosäure aus der Reihe Phenylalanin, Tyrosin, ß-2-Thienylalanin, ß-3-Thienylalanin, 4-Chlorphenylalanin, 0-Methyltyrosin, 0-Benzyltyrosin, 1-Naphthylalanin, 2-Fluorphenylalanin, 3-Fluorphenylalanin oder 4-Fluorphenylalanin bedeutet,
B einen N-terminal mit A und C-terminal mit

$$- NH - \overset{R^2}{\overset{|}{CH}} - \overset{OH}{\overset{|}{CH}} - \overset{R^3}{\overset{|}{CH}} - R^4$$

verknüpften Rest einer Aminosäure aus der Reihe
Phenylalanin, Histidin, Leucin, Asparagin, ß-2-Thienylalanin, ß-3-Thienylalanin, Lysin, Norvalin, 2-Fluorphenylalanin, 3-Fluorphenylalanin, 4-Fluorphenylalanin, Norleucin, S-Methylcystein oder N-Methylhistidin bedeutet,
$R^2$ Isobutyl, Cyclohexylmethyl oder Benzyl bedeutet,
$R^3$ Wasserstoff ist und
$R^4$ für einen Rest der Formel III steht, worin
X abwesend ist,
p 0 ist,
q 0, 1, 2, 3 oder 4 bedeutet und
$R^5$ 2-Pyridyl, 4-Pyridyl-, 2-Imidazolyl oder 1-$(C_1$-$C_4$-Alkyl)-2-imidazolyl bedeutet
sowie deren physiologisch verträglichen Salze.

4. Verfahren zur Herstellung einer Verbindung der Formel I gemäß einem oder mehreren der Ansprüche 1-3, dadurch gekennzeichnet, daß man eine Verbindung der allgemeinen Formel IV

$$H - A - B - NH - \overset{R^2}{\overset{|}{CH}} - \overset{OR^7}{\overset{|}{CH}} - \overset{R^3}{\overset{|}{CH}} - R^4 \qquad (IV)$$

in welcher A, B, $R^2$, $R^3$ und $R^4$ die gleiche Bedeutung wie in Anspruch 1 besitzen und in welcher $R^7$ Wasserstoff oder eine leicht abspaltbare Schutzgruppe bedeutet,

a) mit einem Isocyanat der allgemeinen Formel V

$R^a$ - N = C = O (V),

worin $R^a$ die gleiche Bedeutung wie in Anspruch 1 besitzt, in einem geeigneten organischen Lösungsmittel, oder aber unter Verzicht auf ein Lösungsmittel jeweils wahlweise mit oder ohne Zusatz einer Lewis-Säure oder einer Lewis-Base als Katalysator, bei einer Temperatur zwischen -80 °C und der Siedetemperatur des Lösungsmittels, umsetzt und gegebenenfalls die Schutzgruppe $R^7$ wieder abspaltet, oder

b) nacheinander zunächst mit einem Kohlensäurederivat der allgemeinen Formel VI

$$R^8 - \overset{\overset{\displaystyle O}{\|}}{C} - R^9 \quad (VI),$$

worin $R^8$ und $R^9$ unabhängig voneinander Halogen, ($C_1$-$C_7$)-Alkoxy, ($C_6$-$C_{12}$)-Aryloxy, ($C_1$-$C_7$)-Alkylthio, ($C_6$-$C_{12}$)-Arylthio oder einen Rest Het oder Het-O-bedeuten, wobei Het ein mono- oder bicyclischer Heterocyclus sein kann, oder worin $R^8$ und $R^9$ gemeinsam mit der C=O-Gruppe einem mono- oder bicyclischen Heterocyclus vom Typ

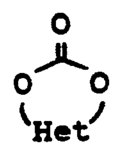

oder

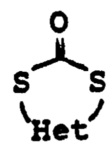

angehören,

vorzugsweise mit Phosgen, 1,1′-Carbonyldiimidazol, 1,1′-Carbonyldi-(1,2,4)-triazol, Di-(N-succinimidyl)-carbonat, Di-(1-benzotriazolyl)-carbonat, N,N′-Carbonyl-bis-(2-methylimidazol) oder 4,6-Diphenylthieno[3,4-d]-1,3-dioxol-2-on-5,5-dioxid (Steglich-Reagens) und anschließend mit einem Amin der allgemeinen Formel VII

$R^a$ - $NH_2$ (VII),

worin $R^a$ die gleiche Bedeutung wie in Anspruch 1 besitzt, in einem inerten organischen Lösungsmittel, mit oder ohne Gegenwart einer Hilfsbase bei einer Temperatur zwischen -80 °C und dem Siedepunkt des jeweiligen Lösungsmittels, umsetzt und gegebenenfalls nach bekannten Methoden die Schutzgruppe $R^7$ sowie gegebenenfalls in den Fragmenten A und B temporär eingeführte Schutzgruppen wieder abspaltet.

5. Verwendung einer Verbindung der Formel I gemäß einem oder mehreren der Ansprüche 1-3 als Heilmittel.

6. Verwendung einer Verbindung der Formel I gemäß einem oder mehreren der Ansprüche 1-3 zur Behandlung des Bluthochdrucks.

7. Pharmazeutisches Mittel enthaltend eine Verbindung der Formel I gemäß einem oder mehreren der Ansprüche 1-3.

Patentansprüche für folgende Vertragsstaaten: ES, GR

1. Verfahren zur Herstellung einer Verbindung der Formel I

$$R^1 - A - B - HN - \overset{R^2}{\overset{|}{CH}} - \overset{OH}{\overset{|}{CH}} - \overset{R^3}{\overset{|}{CH}} - R^4 \qquad (I)$$

in welcher

$R^1$ einen Rest der Formel II bedeutet

$R^a$ - NH - CO - (II)

worin

$R^a$ Wasserstoff, ($C_1$-$C_{10}$)-Alkyl, das gegebenenfalls ein-oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethyloxycarbonylamino substituiert ist,

($C_3$-$C_8$)-Cycloalkyl, ($C_3$-$C_8$)-Cycloalkyl-($C_1$-$C_6$)-alkyl, ($C_6$-$C_{14}$)-Aryl, das gegebenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogen substituiertes Anilino und Trifluormethyl substituiert ist,

($C_6$-$C_{14}$)-Aryl-($C_1$-$C_6$)-alkyl, worin der Arylteil gegebenenenfalls durch einen oder zwei gleiche oder verschiedene Reste aus der Reihe F, Cl, Br, J, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidinomethyl substituiert ist, oder für den Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 S- oder O-Atom auch als Ringglieder steht, der gegebenenfalls durch einen, zwei oder drei gleiche oder verschiedene Reste aus der Reihe F, Cl, Br, Hydroxy, ($C_1$-$C_7$)-Alkoxy, ($C_1$-$C_7$)-Alkyl, ($C_1$-$C_7$)-Alkoxycarbonyl, Amino oder Trifluormethyl substituiert ist,

A einen N-terminal mit $R^1$ und C-terminal mit B verknüpften Rest einer Aminosäure aus der Reihe Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]-thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin und (E)-Dehydrophenylalanin bedeutet,

B einen N-terminal mit A und C-terminal mit

$$NH - \overset{R^2}{\overset{|}{CH}} - \overset{OH}{\overset{|}{CH}} - \overset{R^3}{\overset{|}{CH}} - R^4$$

verknüpften Rest einer Aminosäure, der wie unter A definiert ist, bedeutet,

$R^2$ Wasserstoff, ($C_1$-$C_{10}$)-Alkyl, ($C_4$-$C_7$)-Cycloalkyl, ($C_4$-$C_7$)-Cycloalkyl-($C_1$-$C_4$)-alkyl, ($C_6$-$C_{14}$)-Aryl oder ($C_6$-$C_{14}$)-Aryl-($C_1$-$C_4$)-alkyl bedeutet und

$R^3$ Wasserstoff, ($C_1$-$C_{10}$)-Alkyl, ($C_6$-$C_{14}$)-Aryl oder ($C_6$-$C_{14}$)-Aryl-($C_1$-$C_4$)-alkyl bedeutet,

$R^4$ einen Rest der Formel III bedeutet

$-(CH_2)_p - X - (CH_2)_q - R^5$ (III)

worin X wahlweise abwesend sein kann oder für -O-, -S-, $-CF_2-$, -CO- oder $-CHR^6-$ steht,

p und q unabhängig voneinander 0, 1, 2, 3 oder 4 bedeuten,

$R^6$ Wasserstoff, ($C_1$-$C_7$)-Alkyl, ($C_1$-$C_5$)-Alkoxy, ($C_1$-$C_5$)-Alkylthio, ($C_1$-$C_5$)-Alkylamino-, -OH, $-N_3$, -F, -Cl, -Br oder -I bedeutet, und

$R^5$ Wasserstoff, -OH, $-NH_2$ oder Heteroaryl, das auch teilweise oder vollständig hydriert sein kann, bedeutet sowie deren physiologisch verträgliche Salze dadurch gekennzeichnet, daß man eine Verbindung der allgemeinen Formel IV

$$H - A - B - NH - \overset{R^2}{\overset{|}{CH}} - \overset{OR^7}{\overset{|}{CH}} - \overset{R^3}{\overset{|}{CH}} - R^4 \qquad (IV)$$

in welcher A, B, $R^2$, $R^3$ und $R^4$ die gleiche Bedeutung wie in Anspruch 1 besitzen und in welcher $R^7$ Wasserstoff oder eine leicht abspaltbare Schutzgruppe bedeutet,

a) mit einem Isocyanat der allgemeinen Formel V

$R^a - N = C = O$ (V),

worin $R^a$ die gleiche Bedeutung wie in Anspruch 1 besitzt, in einem geeigneten organischen Lösungsmittel, oder aber unter Verzicht auf ein Lösungsmittel jeweils wahlweise mit oder ohne Zusatz einer Lewis-Säure oder einer Lewis-Base als Katalysator, bei einer Temperatur zwischen -80 °C und der Siedetemperatur des Lösungsmittels, unsetzt und gegebenenfalls die Schutzgruppe $R^7$ wieder abspaltet, oder

b) nacheinander zunächst mit einem Kohlensäurederivat der allgemeinen Formel VI

$$R^8 - \overset{O}{\overset{\|}{C}} - R^9 \quad (VI),$$

worin $R^8$ und $R^9$ unabhängig voneinander Halogen, ($C_1$-$C_7$)-Alkoxy, ($C_6$-$C_{12}$)-Aryloxy, ($C_1$-$C_7$)-Alkylthio, ($C_6$-$C_{12}$)-Arylthio oder einen Rest Het oder Het-O-bedeuten, wobei Het ein mono- oder bicyclischer Heterocyclus sein kann, oder worin $R^8$ und $R^9$ gemeinsam mit der C=O-Gruppe einem mono- oder bicyclischen

Heterocyclus vom Typ

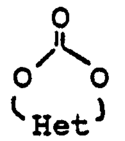 oder 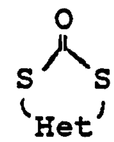

angehören,
vorzugsweise mit Phosgen, 1,1′-Carbonyldiimidazol, 1,1′-Carbonyldi-(1,2,4)-triazol, Di-(N-succinimidyl)-carbonat, Di-(1-benzotriazolyl)-carbonat, N,N′-Carbonyl-bis-(2-methylimidazol) oder 4,6-Diphenylthieno[3,4-d]-1,3-dioxol-2-on-5,5-dioxid (Steglich-Reagens) und anschließend mit einem Amin der allgemeinen Formel VII
$R^a - NH_2$ (VII),
worin $R^a$ die gleiche Bedeutung wie in Anspruch 1 besitzt, in einem inerten organischen Lösungsmittel, mit oder ohne Gegenwart einer Hilfsbase bei einer Temperatur zwischen -80 °C und dem Siedepunkt des jeweiligen Lösungsmittels, umsetzt und gegebenenfalls nach bekannten Methoden die Schutzgruppe $R^7$ sowie gegebenenfalls in den Fragmenten A und B temporär eingeführte Schutzgruppen wieder abspaltet.

2. Verfahren gemäß Anspruch 1, dadurch gekennzeichnet, daß
$R^1$ einen Rest der Formel II bedeutet, worin
$R^a$ Wasserstoff, $(C_1-C_{10})$-Alkyl, $(C_1-C_4)$-Alkoxy-$(C_1-C_{10})$-alkyl, Carboxy-$(C_1-C_{10})$-alkyl, $(C_1-C_4)$-Alkoxycarbonyl, $(C_1-C_{10})$-alkyl, Amino-$(C_1-C_{10})$-alkyl, Diamino-$(C_1-C_{10})$-alkyl, $(C_1-C_4)$-Alkylamino-$(C_1-C_{10})$-alkyl, Di$(C_1-C_4)$alkylamino-$(C_1-C_{10})$-alkyl, N,N′-Di-$(C_1-C_4)$-alkyldiamino-$(C_1-C_{10})$-alkyl, N,N,N′,N-Tetra$(C_1-C_4)$-alkyldiamino-$(C_1-C_{10})$-alkyl, $(C_1-C_4)$-Alkoxycarbonylamino-$(C_1-C_{10})$-alkyl, $(C_7-C_{15})$-Aralkoxycarbonylamino-$(C_1-C_{10})$-alkyl, Cyclopentyl, Cyclohexyl, Cycloheptyl, Cyclopentyl-$(C_1-C_6)$-alkyl, Cyclohexyl-$(C_1-C_6)$-alkyl, Phenyl, das gegebenenfalls durch einen oder zwei gleiche oder verschiedene Reste aus der Reihe F, Cl, Br, J, Hydroxy, $(C_1-C_4)$-Alkoxy, $(C_1-C_4)$-Alkyl, $(C_1-C_4)$-Alkoxycarbonyl, Amino oder Trifluormethyl substituiert ist, $(C_6-C_{10})$-Aryl-$(C_1-C_6)$-alkyl, worin der Arylteil wie oben Phenyl substituiert sein kann, bedeutet oder für den Rest eines 5- oder 6-gliedrigen monocyclischen oder 9- oder 10-gliedrigen bicyclischen Heteroaromaten mit mindestens 1 C-Atom, 1-4 N-Atomen und/oder 1 S- oder O-Atom auch als Ringglieder Steht, der gegebenenfalls durch einen, zwei oder drei gleiche oder verschiedene Reste aus der Reihe F, Cl, Br, Hydroxy, $(C_1-C_7)$-Alkoxy, $(C_1-C_7)$-Alkyl, $(C_1-C_7)$-Alkoxycarbonyl, Amino oder Trifluormethyl substituiert ist,
A einen N-terminal mit $R^1$ und C-terminal mit B verknüpften Rest einer Aminosäure aus der Reihe Phenylalanin, Histidin, Tyrosin, Tryptophan, ß-2-Thienylalanin, ß-3-Thienylalanin, ß-2-Furylalanin, ß-3-Furylalanin, 4-Chlorphenylalanin, 2-Pyridylalanin, 3-Pyridylalanin, Cyclohexylalanin, Cyclohexylglycin, im-Methylhistidin, O-Methyltyrosin, O-Benzyltyrosin, O-tert.Butyltyrosin, Phenylglycin, 1-Naphthylalanin, 2-Naphthylalanin, 4-Nitrophenylalanin, 2-Fluorphenylalanin, 3-Fluorphenylalanin, 4-Fluorphenylalanin, Homophenylalanin, DOPA, O-Dimethyl-DOPA, 2-Amino-4-(2-thienyl)-buttersäure, Benzodioxol-5-yl-alanin, N-Methylhistidin, 2-Amino-4-(3-thienyl)-buttersäure, 3-(2-Thienyl)-serin, (Z)-Dehydrophenylalanin oder (E)-Dehydrophenylalanin bedeutet,
B einen N-terminal mit A und C-terminal mit

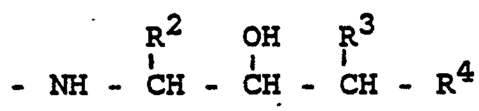

verknüpften Rest einer Aminosäure aus der Reihe
Phenylalanin, Histidin, Tyrosin, Tryptophan, Methionin, Leucin, Isoleucin, Asparagin, Asparaginsäure, ß-2-Thienylalanin, ß-3-Thienylalanin, ß-2-Furylalanin, ß-3-Furylalanin, Lysin, Ornithin, Valin, Alanin, 2,4-Diaminobuttersäure, Arginin, 4-Chlorphenylalanin, Methioninsulfon, Methioninsulfoxid, 2-Pyridylalanin, 3-Pyridylalanin, Cyclohexylalanin, Cyclohexylglycin, im-Methylhistidin, O-Methyltyrosin, O-Benzyltyrosin, O-tert.-Butyltyrosin, Phenylglycin, 1-Naphthylalanin, 2-Naphthylalanin, 4-Nitrophenylalanin, Norvalin, ß-2-Benzo[b]thienylalanin, ß-3-Benzo[b]thienylalanin, 2-Fluorphenylalanin, 3-Fluorphenylalanin, 4-Fluorphenylalanin, Norleucin, Cystein, S-Methyl-Cystein, 1,2,3,4-Tetrahydroisochinolin-3-carbonsäure, Homophenylalanin, DOPA, O-Dimethyl-DOPA, 2-Amino-4-(2-thienyl)-buttersäure, Benzodioxol-5-yl-alanin, N-Methyl-histidin, 2-Amino-4-(3-thienyl)-buttersäure, 3-(2- Thienyl)-serin (Z)-Dehydrophenylalanin und (E)-Dehydrophenylalanin, bedeutet,
$R^2$ Wasserstoff, $(C_1-C_{10})$-Alkyl, $(C_4-C_7)$-Cycloalkyl, $(C_4-C_7)$-Cycloalkyl-$(C_1-C_4)$-alkyl, $(C_6-C_{14})$-Aryl oder $(C_6-C_{14})$-alkyl bedeutet,

$R^3$ Wasserstoff, $(C_1\text{-}C_{10})$-Alkyl, $(C_6\text{-}C_{14})$-Aryl oder $(C_6\text{-}C_{14})$-Aryl-$(C_1\text{-}C_4)$-alkyl bedeutet,
$R^4$ einen Rest der Formel III bedeutet, worin
X wahlweise abwesend sein kann oder für -O-, -S-, $-CF_2-$, -CO- oder $-CHR^6-$ steht,
p und q unabhängig voneinander 0, 1, 2, 3 oder 4 bedeuten,
$R^6$ Wasserstoff, $(C_1\text{-}C_7)$-Alkyl, $(C_1\text{-}C_5)$-Alkoxy, $(C_1\text{-}C_5)$-Alkylthio, $(C_1\text{-}C_5)$-Alkylamino, -OH, $-N_3$, -F, -Cl, -Br oder -I bedeutet, und
$R^5$ Wasserstoff, -OH, $-NH_2$ oder Heteroaryl, das auch teilweise oder vollständig hydriert sein kann, bedeutet

3. Verfahren gemäß einem oder mehreren der Ansprüche 1-2, dadurch gekennzeichnet, daß
$R^1$ einen Rest der Formel II bedeutet, worin
$R^a$ Wasserstoff, $(C_1\text{-}C_6)$-Alkyl, Carboxy-$(C_1\text{-}C_6)$-alkyl, Amino-$(C_1\text{-}C_6)$-alkyl, Diamino-$(C_1\text{-}C_6)$-alkyl, $(C_1\text{-}C_4)$-Alkylamino-$(C_1\text{-}C_6)$-alkyl, Di-$(C_1\text{-}C_4)$-alkylamino-$(C_1\text{-}C_6)$-alkyl, N,N′-Di-$(C_1\text{-}C_4)$-alkyldiamino-$(C_1\text{-}C_6)$-alkyl, N,N,N′,N′-Tetra-$(C_1\text{-}C_4)$-alkyldiamino-$(C_1\text{-}C_6)$-alkyl, $(C_1\text{-}C_4)$-Alkoxycarbonylamino-$(C_1\text{-}C_6)$-alkyl oder Phenyl, das gegebenenfalls durch einen oder zwei gleiche oder verschiedene Reste aus der Reihe F, Cl, Hydroxy oder Amino substituiert ist, bedeutet,
A einen N-terminal mit $R^1$ und C-terminal mit B verknüpften Rest einer Aminosäure aus der Reihe Phenylalanin, Tyrosin, ß-2-Thienylalanin, ß-3-Thienylalanin, 4-Chlorphenylalanin, 0-Methyltyrosin, 0-Benzyltyrosin, 1-Naphthylalanin, 2-Fluorphenylalanin, 3-Fluorphenylalanin oder 4-Fluorphenylalanin bedeutet,
B einen N-terminal mit A und C-terminal mit

$$-NH-\underset{|}{\overset{R^2}{}}\!\!CH-\overset{OH}{\underset{}{CH}}-\overset{R^3}{CH}-R^4$$

verknüpften Rest einer Aminosäure aus der Reihe
Phenylalanin, Histidin, Leucin, Asparagin, ß-2-Thienylalanin, ß-3-Thienylalanin, Lysin, Norvalin-2-Fluorphenylalanin, 3-Fluorphenylalanin, 4-Fluorphenylalanin, Norleucin, S-Methylcystein oder N-Methylhistidin bedeutet,
$R^2$ Isobutyl, Cyclohexylmethyl oder Benzyl bedeutet,
$R^3$ Wasserstoff ist und
$R^4$ für einen Rest der Formel III steht, worin
X anwesend ist,
p 0 ist,
q 0, 1, 2, 3 oder 4 bedeutet und
$R^5$ 2-Pyridyl, 4-Pyridyl-, 2-Imidazolyl oder 1-$(C_1\text{-}C_4$-Alkyl)-2-imidazolyl bedeutet.

4. Verwendung einer nach einem der Verfahren der Ansprüche 1 bis 3 hergestellten Verbindung der Formel I als Heilmittel.

5. Verwendung einer nach einem der Verfahren der Ansprüche 1 bis 3 hergestellten Verbindung der Formel I bei der Behandlung des Bluthochdrucks.

6. Verfahren zur Herstellung einer pharmazeutischen Zubereitung enthaltend eine nach einem der Verfahren der Ansprüche 1 bis 3 hergestellten Verbindung der Formel I, dadurch gekennzeichnet, daß man diese zusammen mit einem physiologisch unbedenklichen Träger und gegebenenfalls weiteren Hilfs- und Zusatzstoffen in eine geeignete Darreichungsform bringt.